# EUROPÄISCHE PATENTSCHRIFT

(11) **EP 3 839 571 B1**
(45) Veröffentlichungstag und Bekanntmachung des Hinweises auf die Patenterteilung: **05.10.2022**
(21) Anmeldenummer: 19218975.1
(22) Anmeldetag: 20.12.2019
(51) Int. Cl.: G01T 1/10

(54) **STOFFVERBUND ZUM DETEKTIEREN FREIER NEUTRONEN MIT EINER EFFEKTIVEN KERNLADUNGSZAHL ÄHNLICH MENSCHLICHEM KÖRPERGEWEBE DURCH NUTZUNG VON BERYLLIUMOXID UND LITHIUMTETRABORAT; DOSIMETER; VERFAHREN ZUM ERFASSEN BEZIEHUNGSWEISE DETEKTIEREN FREIER NEUTRONEN**
MATERIAL COMPOSITE FOR DETECTING FREE NEUTRONS WITH AN EFFECTIVE CORE CHARGE NUMBER SIMILAR TO HUMAN BODY TISSUE BY USING BERYLLIUM OXIDE AND LITHIUM TETRABORATE; DOSIMETER; METHOD FOR DETECTING FREE NEUTRONS
MATÉRIAU COMPOSITE PERMETTANT DE DÉTECTER LE NEUTRON LIBRE À UN NOMBRE ATOMIQUE EFFECTIF SIMILAIRE AU TISSU CORPOREL HUMAIN EN UTILISANT DE L'OXYDE DE BÉRYLLIUM ET/OU DU TÉTRABORATE DE LITHIUM ; DOSIMÈTRE ; PROCÉDÉ DE DÉTERMINATION ET DE DÉTECTION DU NEUTRON LIBRE

(43) Veröffentlichungstag der Anmeldung: 23.06.2021
(73) Patentinhaber: Mirion Technologies (AWST) GmbH, 81739 München (DE)
(72) Erfinder: Scheubert, Peter Georg, 83714 Miesbach (DE); Kormoll, Thomas, 01139 Dresden (DE)
(74) Vertreter: Hofstetter, Schurack & Partner

(56) Entgegenhaltungen:
- US-A- 3 449 136
- K. SAKASAI ET AL: "RBPO/sub 5/ (R=Ca, Sr)-based storage phosphors for neutron detection", IEEE TRANSACTIONS ON NUCLEAR SCIENCE., Bd. 52, Nr. 5, 1. Oktober 2005 (2005-10-01), Seiten 1856-1859, XP055698851, US ISSN: 0018-9499, DOI: 10.1109/TNS.2005.856617
- YUKIHARA E G ET AL: "Development of new optically stimulated luminescence (OSL) neutron dosimeters", RADIATION MEASUREMENTS, ELSEVIER, AMSTERDAM, NL, Bd. 43, Nr. 2-6, 1. Februar 2008 (2008-02-01), Seiten 309-314, XP022733809, ISSN: 1350-4487, DOI: 10.1016/J.RADMEAS.2007.10.005 [gefunden am 2008-02-01]
- KRAUS W ET AL: "Entwicklungstendenzen der Personendosimetrie beruflich strahlenexponierter Personen", KERNENERGIE,, Bd. 27, Nr. 8, 1. August 1984 (1984-08-01) , Seiten 325-334, XP001431559,
- YUKIHARA E G ET AL: "Systematic development of new thermoluminescence and optically stimulated luminescence materials", JOURNAL OF LUMINESCENCE, Bd. 133, 2013, Seiten 203-210, XP028957785, ISSN: 0022-2313, DOI: 10.1016/J.JLUMIN.2011.12.018

## Beschreibung

Die vorliegende Erfindung betrifft einen Stoffverbund zum Detektieren freier Neutronen mit einem Konvertermaterial, welches dazu ausgebildet ist, in Folge eines Einfangs von Neutronen eine Sekundärstrahlung zu erzeugen und einem Detektormaterial, welches dazu ausgebildet ist, eine Information bezüglich einer Quantität der Sekundärstrahlung zu speichern und bei einer späteren Auswertung mittels optisch stimulierter Lumineszenz wieder freizugeben, wobei das Konvertermaterial und das Detektormaterial jeweils aus einer Vielzahl von Partikeln bestehen, welche gemeinsam in dem Stoffverbund als Stoffgemenge vorliegen. Außerdem betrifft die Erfindung ein Dosimeter, welches einen solchen Stoffverbund aufweist. Zuletzt betrifft die Erfindung ein Verfahren zum Detektieren freier Neutronen mittels eines Stoffverbunds.

Zum Messen beziehungsweise Detektieren von Strahlung, insbesondere ionisierender Strahlung, sind unterschiedliche Arten an Dosimetern bekannt. Für Messgeräte zur Ermittlung kumulierter Strahlungsdosen ist die Bezeichnung Dosimeter gebräuchlich. Eine preisgünstige Realisierungsvariante bei dennoch hoher Genauigkeit stellt der Einsatz passiver Dosimeter dar. Passive Dosimeter zeichnen sich dadurch aus, dass die vom Dosimeter absorbierte Strahlungsenergie zeitinvariant und dauerhaft in der Struktur des Detektormaterials gespeichert wird. Die durch ionisierende Strahlung im Detektor erzeugten freien Ladungsträger können teilweise direkt als Informationsspeicher für die Information dienen. Insbesondere können im Detektormaterial mittels ionisierender Prozesse erzeugte freie Elektronen auf Energieniveaus gelangen, die einerseits ein gegenüber dem Grundzustand angehobenes Energieniveau aufweisen aber bei Raumtemperatur nicht in den Grundzustand oder höhere Anregungszustände gelangen können. Diese Elektronen sind somit auf einem solchen Energieniveau sprichwörtlich gefangen, die englische Bezeichnung für entsprechende Energieniveaus lautet Traps.

Lange Zeit war es üblich, derartige passive Dosimeter mit einem Detektormaterial auszustatten, welches dem Prinzip der Thermolumineszenzdosimetrie (TLD) ausgewertet werden kann. Beim Auslesen werden Elektronen durch thermische Stimulation (Erwärmung des Detektormaterials) aus den Energieniveaus beziehungsweise Traps befreit, in welchen sie gefangen wurden. Hierdurch gelangen die entsprechenden Elektronen aus den Traps typischerweise zu höheren Energien ins Leitungsband des Detektormaterials. Bei einer nachfolgenden Rekombination mit den zugehörigen Löchern wird Licht einer spezifischen Wellenlänge abgegeben. Aus der Intensität des Lichts beziehungsweise der Photonenzahl des Lichts kann auf die kumulierte Strahlungsexposition des Detektors und somit die absorbierte Strahlendosis geschlossen werden. Insbesondere ist bei geeigneten Detektormaterialien die Intensität beziehungsweise Photonenzahl des Lumineszenzlichtes über weite Bereiche proportional zur absorbierten Strahlendosis.

Als moderneres Verfahren für die Personendosimetrie hat sich zunehmend die optisch stimulierte Lumineszenz (OSL) durchgesetzt. Die Speicherung der Dosisinformation erfolgt analog zu TLD ebenfalls in stabilen Traps, d.h. Energieniveaus zwischen Valenz- und Leitungsband des Detektormaterials. Die Freigabe der gefangenen Elektronen, die in analoger Weise wie der TL Prozess die Zufuhr von Energie erfordert, erfolgt hierbei jedoch durch eine Beleuchtung des entsprechenden Detektormaterials mit Licht. Vorteil der Nutzung optisch stimulierter Lumineszenz ist, dass Lichtleistung zu einer unmittelbaren und taktbaren Energiezufuhr verwendet werden kann, während die Erwärmung des Detektors Zeit und ggf. aufwändige Vorrichtungen erfordert, um einen definierten Heizprozess garantieren zu können. Ferner zeichnen sich für OSL-Dosimetrie geeignete Materialen durch Zeitinvarianz der gespeicherten Information aus. Ein Prozess, der zum Verlust gespeicherter Information führt, wird als Fading bezeichnet. In Materialien, bei denen Fading stattfindet, können bereits bei Raumtemperatur Elektronen von dem Energieniveau ihres Traps auf ein normales Energieniveau zurückfallen und stehen nicht mehr als Informationsträger zur Verfügung. Hieraus folgt eine Verfälschung des Messergebnisses und somit große, zeitabhängige Fehler beim anschließenden Bestimmen der Strahlendosis.

Häufig ist auch die Messung von Neutronenstrahlung von Interesse. Detektormaterialien, welche nach dem Prinzip der optisch stimulierten Lumineszenz arbeiten, reagieren häufig nicht auf Neutronenstrahlung, weil die im Detektor vorhandenen Isotope keine nennenswert großen Einfangquerschnitte für Neutronen besitzen und deshalb nicht direkt mit dem Strahlungsfeld wechselwirken. Abhilfe schaffen so genannte Konvertermaterialien, welche Neutronen einfangen können. Typischerweise folgt einem Neutroneneinfang zeitnah der radioaktive Zerfall des entstandenen Isotops d.h. die Konvertermaterialien geben Sekundärstrahlung ab. Diese Sekundärstrahlung ist wiederum ionisierend und durch das Detektormaterial erfassbar.

Aus der US 2013/00 15 339 A1 ist beispielsweise ein Gerät zum Messen von Strahlung in Bohrlöchern geologischer Formationen bekannt. Eine Messanordnung des Geräts weist dabei ein Material auf, welches Auswertung durch optisch stimulierte Lumineszenz ermöglicht. Die Messanordnung kann weiterhin eine Konversionslage aufweisen, um nicht-ionisierende Strahlung, beispielsweise Neutronen, in ionisierende Strahlung umzuwandeln.

Die Veröffentlichung "Development of new optically stimulated luminescence (OSL) neutron dosimeters" in RADIATION MEASUREMENTS, ELSEVIER, AMSTERDAM, NL, Bd. 43, Nr. 2-6, 1. Feb 2008, S. 309-314 von E.G. Yukihara et. al. schlägt die Durchmengung von Aluminiumoxid (AL₂O₃) als Detektormaterial und einem Konvertermaterial zur Erfassung von Neutronen vor.

Aus der US 3 449 136 A ist die Nutzung von Berylliumoxid (BeO) im Bereich der Detektion thermischer Neutronen bekannt.

Die Veröffentlichung "RBPO5 (R=Ca, Sr)-Based Storage Phosphors for Neutron Detection" in IEEE TRANSACTIONS ON NUCLEAR SCIENCE, Bd. 52, Nr. 5, 1. Okt. 2008, S. 1856-1859 von K. Sakasai et al offenbart eine Verbesserung von Ergebnissen einer photostimulierten Lumineszenz von SrBPO₅:Eu²⁺-Phosporen durch eine Zugabe von Li₂B₄O₇.

Es ist Aufgabe der vorliegenden Erfindung, die Detektion von Neutronen im Hinblick auf die Anwendung in der Personendosimetrie, also die Abschätzung einer durch einen Menschen absorbierten Dosis, zu verbessern.

Diese Aufgabe wird erfindungsgemäß gelöst durch die Gegenstände der unabhängigen Patentansprüche. Vorteilhafte Ausführungsformen und zweckmäßige Weiterbildungen der Erfindung sind Gegenstand der Unteransprüche.

Die vorliegende Erfindung beruht auf der Idee, dass eine effektive Kernladungszahl des im Dosimeter verwendeten Detektormaterials auch auf die Neutronendosimetrie einen großen Einfluss haben kann. So werden zwar Neutronen durch die Kernladung von Atomen nur geringfügig beeinflusst, doch bereits hier kann sich die Genauigkeit einer Dosisbestimmung verbessern, je näher die effektive Kernladungszahl eines für die passive Dosimetrie verwendeten Stoffverbunds aus Konvertermaterials und Detektormaterial an der effektiven Kernladungszahl menschlichen Gewebes liegt. Weitere Vorteile ergeben sich zudem dann, wenn gleichzeitig mit einer Neutronendosis auch eine Dosis ionisierender Strahlung bestimmt werden soll, die typischerweise gleichzeitig mit den Neutronen auftritt. Die Bestimmung eines Referenzwerts für ionisierende Strahlung z.B. Gammastrahlung ist nötig, da Neutronendosimeter den reinen Neutronenanteil der Strahlung quantifizieren sollen, d.h. ein zum gleichen Zeitpunkt auftretender Anteil anderer Strahlungsarten kompensiert werden muss. Die präzise Bestimmung der Neutronendosis erfordert daher üblicherweise die Ermittlung eines Referenzwerts für gleichzeitig einfallende Photonenstrahlung, der nachgelagert subtrahiert wird, um die Neutronendosis zu bestimmen. Durch eine erhöhte Genauigkeit des Referenzwerts kann daher auch die Genauigkeit beim Bestimmen der Neutronendosis verbessert werden.

Von diesen grundlegenden Überlegungen ausgehend stellt die Erfindung einen Stoffverbund zum Detektieren freier Neutronen bereit, der hinsichtlich seiner effektiven Kernladungszahl gegenüber etablierten TL oder OSL Detektormaterialen verbessert und der Kernladungszahl des menschlichen Gewebes angeglichen ist.

Bei den Strahlungsarten, zu deren Detektion der Stoffverbund direkt nutzbar sein soll beziehungsweise zu deren Einfangen das Konvertermaterial ausgebildet ist, handelt es sich insbesondere um thermische Neutronen. Alternativ oder zusätzlich können auch schnelle Neutronen detektiert werden. Insbesondere ist der Stoffverbund zur Detektion von Neutronenstrahlung ausgebildet. Da Einfangquerschnitte für Neutronen mit zunehmender Energie drastisch abnehmen, kann es zur Detektion schneller Neutronen sinnvoll sein, vor dem Eintreffen im Detektormaterial deren Geschwindigkeit zu reduzieren, d.h. sie zu moderieren. Zum Detektieren schneller Neutronen kann ein Dosimeter, in welchem der Stoffverbund eingesetzt ist, zusätzlich zum Stoffverbund ein Moderatormaterial aufweisen. Alternativ oder zusätzlich kann der Stoffverbund um ein solches Moderatormaterial erweitert sein. Es ist alternativ oder zusätzlich möglich, dass das Konvertermaterial und/oder das Detektormaterial als Moderatormaterial wirken. Das Moderatormaterial zeichnet sich dadurch aus, dass es geeignet ist, schnelle Neutronen abzubremsen. Mit anderen Worten werden schnelle Neutronen durch das Moderatormaterial in ihrer kinetischen Energie reduziert. Durch die Reduzierung der kinetischen Energie beziehungsweise durch das Abbremsen der schnellen Neutronen können diese in einen Zustand überführt werden, in welchem diese mit einem größeren Einfangquerschnitt eingefangen werden können. Bei thermischen Neutronen handelt es sich um freie Neutronen mit einer kinetischen Energie von etwa 25 Millielektronenvolt. Es kann vorgesehen sein, dass das Konvertermaterial neben thermischen Neutronen auch schnelle Neutronen mit ausreichendem Einfangquerschnitt einfängt. In diesem Fall könnte auf das Moderatormaterial zum Abbremsen schneller Neutronen verzichtet werden. Für viele Anwendungsfälle ist die Detektion schneller Neutronen unerheblich, für derartige Anwendungsfälle kann auf ein Moderatormaterial verzichtet werden und es ist zudem nicht nötig, dass das Konvertermaterial schnelle Neutronen mit großem Einfangquerschnitt einfangen kann.

Wie eingangs bereits erwähnt wurde, ist das Konvertermaterial dazu ausgebildet, Neutronen einzufangen und auf ein solches Einfangen hin eine Sekundärstrahlung zu erzeugen. Bei der Sekundärstrahlung handelt es sich insbesondere um eine andere Strahlungsform als Neutronenstrahlung. Beispielsweise handelt es sich bei der Sekundärstrahlung ionisierende Strahlung, beispielsweise um Photonen, insbesondere Röntgen- oder Gammastrahlung, oder energiereiche Teilchen wie Betastrahlung, Alphastrahlung, Tritiumstrahlung oder ähnliches. Im Allgemeinen kann es sich bei der Sekundärstrahlung bevorzugt um ionisierende Strahlung handeln. Jedenfalls ist das Konvertermaterial derart zu wählen, dass dieses auf den Einfang der Neutronen hin eine geeignete Sekundärstrahlung erzeugt, welche mittels des Detektormaterials quantifiziert werden kann.

Das Detektormaterial ist dazu ausgebildet, auf einen Einfang der Sekundärstrahlung hin eine Information bezüglich der Sekundärstrahlung zu speichern. Die Auswertung der Information erfolgt insbesondere mittels des Prinzips optisch stimulierter Lumineszenz (OSL). Deren Prinzip wurde einleitend bereits dargelegt und wird daher hier nicht erneut beschrieben. Somit ist das Detektormaterial dazu ausgebildet, die Information mittels eines Prozesses zu speichern, welcher der späteren Auswertung mittels des Prinzips optisch stimulierter Lumineszenz (OSL) zugänglich ist. Beispielsweise ist das Detektormaterial dazu ausgebildet, die Information auf eine Absorption, eine Streuung oder einen (unelastischen) Stoß mit einem Photon, Elektron, Tritiumkern oder Heliumkern der Sekundärstrahlung hin zu speichern. Die Durchdringung mit Sekundärstrahlung ist insbesondere dosisproportional bezogen auf die Anzahl der eingefangenen freien Neutronen. Vorteilhafterweise ist vorgesehen, dass das Detektormaterial dazu ausgebildet ist, die gespeicherte Information bei einer späteren Auswertung dosisproportional in Form eines Emissionsspektrums bzw. von Lumineszenzlicht abzugeben.

Im Rahmen der späteren Auswertung wird das Detektormaterial insbesondere mit einem Stimulationsspektrum, insbesondere monochromatischem Licht einer bestimmten Wellenlänge, beleuchtet und somit stimuliert. Als Folge der Beleuchtung beziehungsweise Stimulation gibt das Detektormaterial die gespeicherte Information mittels eines Emissionsspektrum ab. Dabei muss das typischerweise deutlich intensivere Stimulationslicht des Stimulationsspektrums mittels geeigneter optischer Filter von dem im Falle niedriger Strahlungsdosen sehr schwachen Lumineszenzlicht des Emissionsspektrums getrennt werden muss was nur aufgrund eines Wellenlängenunterschieds möglich ist. Bei der Mischung unterschiedlicher Detektormaterialien ist damit zu rechnen, dass die Emission des Lumineszenzlichtes bei unterschiedlichen Welllängen erfolgt. Dies bedeutet, dass auf Basis des Antwortsignals und seiner Wellenlänge bzw. durch die Wahl unterschiedlicher Stimulationsspektren, insbesondere mit monochromatischem Licht einer unterschiedlichen Wellenlänge, eine getrennte oder sequentielle Auswertung der unterschiedlichen Detektormaterialien möglich sein kann.

Es ist vorteilhaft, die Detektoreinheit als Stoffverbund d.h. aus einem Stoffgemenge bestehend aus jeweils fein zerteiltem Konvertermaterial und Detektormaterial zu gestalten. In dem Stoffgemenge sind das Konvertermaterial und das Detektormaterial zumindest im Wesentlichen vollständig durchmengt beziehungsweise durchmischt. Es soll sichergestellt werden, dass sich in der Nähe jedes Partikels mit Konvertermaterial in direkter Nachbarschaft Partikel mit Detektormaterial befinden. Auf diese Weise kann eine Empfindlichkeit des Stoffverbunds maximiert werden. Die Sekundärstrahlung muss nur eine möglichst geringe Weglänge von dem Konvertermaterial zu dem Detektormaterial zurücklegen. Auf diese Weise wird eine unerwünschte anderweitige Absorption der Sekundärstrahlung, z.B. im Konvertermaterial selbst begrenzt.

Zur Lösung der oben genannten Aufgabe beziehungsweise zur Angleichung der effektiven Kernladungszahl des Stoffverbunds an die effektive Kernladungszahl menschlichen Gewebes ist es erfindungsgemäß vorgesehen, das Detektormaterial und das Konvertermaterial aus einem jeweiligen Material zu bilden, welches jeweils eine entsprechend effektive Kernladungszahl aufweist. Dies ist erfindungsgemäß der Fall für Berylliumoxid (BeO) als Detektormaterial sowie Lithiumtetraborat (Li₂B₄O₇) als Konvertermaterial , wenn die Lithium-Atome zumindest teilweise, insbesondere zu einem nennenswerten Anteil von beispielsweise zumindest 5%, zumindest 10%, zumindest 20% oder zumindest 30%, aus dem Isotop ⁶Li gebildet sind. Zusätzlich eignet sich Lithiumtetraborat auch als Detektormaterial.

Ein erster Aspekt der vorliegenden Erfindung betrifft somit einen Stoffverbund zum Detektieren freier Neutronen, mit einem Konvertermaterial, welches dazu ausgebildet ist, als Folge eines Neutroneneinfangs eine Sekundärstrahlung zu erzeugen, und einem Detektormaterial, welches dazu ausgebildet ist, eine Information bezüglich einer Quantität der Sekundärstrahlung zu speichern und bei einer späteren Auswertung mittels optisch stimulierter Lumineszenz wieder freizugeben, wobei das Konvertermaterial und das Detektormaterial jeweils in einer Vielzahl an Partikeln vorliegen, welche gemeinsam in dem Stoffverbund als Stoffgemenge vorliegen.

Erfindungsgemäß ist vorgesehen, dass das Detektormaterial aus Berylliumoxid und das Konvertermaterial aus Lithiumtetraborat gebildet sind. Mit anderen Worten weist der Stoffverbund Berylliumoxid als Detektormaterial und Lithiumtetraborat als Konvertermaterial auf. Selbstverständlich gelten die Weiterbildungen, welche in Bezug auf die Nutzung von Lithiumtetraborat als Konvertermaterial ausgeführt wurden, auch für diese Ausführungsform analog. Insbesondere kann auch bei einer Nutzung von Berylliumoxid als Detektormaterial und Lithiumtetraborat als Konvertermaterial vorgesehen sein, dass die Isotope ⁶Li und/oder ¹⁰B in dem Lithiumtetraborat gegenüber ihrer natürlichen Häufigkeit angereichert sind. Auch gilt für die vorliegende Kombination aus Lithiumtetraborat und Berylliumoxid, dass eine zweistufige Auswertung mittels optisch stimulierter Lumineszenz möglich ist. Dies gilt insbesondere daher, da die jeweiligen Stimulationsspektren und Emissionsspektren von Berylliumoxid und Lithiumtetraborat unterschiedlich sind. Insofern gelten die in den genannten Kontexten offenbarten Vorteile hier analog. Als zusätzlicher Vorteil ergibt sich, dass bei der Nutzung von Berylliumoxid und Lithiumtetraborat eine effektive Kernladungszahl erzielt wird, welche nur eine geringe Abweichung von der effektiven Kernladungszahl menschlichen Gewebes aufweist, da dies bereits für die effektive Kernladungszahl beider Bestandteile, Berylliumoxid und Lithiumtetraborat, einzeln gilt.

Der jeweilige Stoffverbund ist in seiner Zusammensetzung nicht beschränkt auf das Konvertermaterial und das Detektormaterial. Der Stoffverbund kann zusätzlich noch weitere Materialien, wie beispielsweise das oben genannte Moderatormaterial oder Bindemittel aufweisen. Wie eingangs beschrieben wurde, kann durch Annäherung der effektiven Kernladungszahl des Stoffverbunds in Richtung der effektiven Kernladungszahl menschlichen Gewebes die Effizienz und/oder die Genauigkeit und/oder Zuverlässigkeit des Stoffverbunds im Rahmen einer Personendosimetrie verbessert werden. Da die genannten Materialien Berylliumoxid und Lithiumtetraborat jeweils für sich schon die Gleichheit der effektiven Kernladungszahl des Körpergewebes mit sich bringen, sind diese gleichermaßen geeignet mit demselben erfinderischen Gedanken die genannte Aufgabe zu lösen.

Gemäß einer Weiterbildung des Stoffverbunds ist vorgesehen, dass bei dem Lithiumtetraborat die Isotope ⁶Li und/oder ¹⁰B gegenüber ihrer natürlichen Häufigkeit angereichert sind. Mit anderen Worten kann bei dem im Lithiumtetraborat enthaltenen Lithium das Lithiumisotop mit der Nukleonenzahl 6 gegenüber der natürlichen Häufigkeit des Lithiumisotops angereichert sein. Alternativ oder zusätzlich kann bei dem im Lithiumtetraborat enthaltenen Bor das Bor-Isotop mit der Nukleonenzahl 10 gegenüber der natürlichen Häufigkeit des Borisotops angereichert sein. Die Isotope ⁶Li und ¹⁰B weisen jeweils einen deutlich größeren Einfangquerschnitt für Neutronen auf, als die übrigen Isotope des jeweiligen Elements. Somit kann durch eine entsprechende Anreicherung der Einfangquerschnitt des Lithiumtetraborats für Neutronen erhöht beziehungsweise verbessert werden. Auf diese Weise kann ein größerer Anteil der auf das Konvertermaterial treffenden freien Neutronen eingefangen werden. In der Konsequenz wird hierdurch die Quantität der erzeugten Sekundärstrahlung erhöht, da deren Emission proportional dem Einfang von Neutronen erfolgt. Insgesamt kann eine Effektivität und auch eine Genauigkeit des Stoffverbunds zum Detektieren von Neutronen somit verbessert werden.

Da das Detektormaterial aus einem anderen Material als Lithiumtetraborat gebildet ist, zeigt sich vor diesem Hintergrund, dass in diesem Fall zwei Materialien, welche als Detektormaterial nutzbar sind, Teil des Stoffverbunds sind. Mit anderen Worten enthält der Stoffverbund einerseits das Lithiumtetraborat als Detektormaterial und zusätzlich Berylliumoxid als Detektormaterial. Auf diese Weise ist der Stoffverbund einer zweistufigen Auswertung mittels optisch stimulierter Lumineszenz zugänglich. Besonders vorteilhaft kann das Emissionsspektrum des Detektormaterials von dem Emissionsspektrum des Lithiumtetraborats getrennt werden. Auf diese Weise ist der Stoffverbund für eine doppelte Auswertung mittels optisch stimulierter Lumineszenz mit jeweils unterschiedlichen Stimulationsspektren und Emissionsspektren geeignet. Die mehrfache Auswertbarkeit schlägt sich üblicherweise in erhöhter Genauigkeit der Messung nieder.

Gemäß einer Weiterbildung der Erfindung ist vorgesehen, dass die Anteile an dem Konvertermaterial und dem Detektormaterial in dem Stoffverbund derart gewählt sind, dass sich eine effektive Kernladungszahl zwischen 6,1 und 8,1, vorzugsweise zwischen 6,7 und 7,5, ergibt. Dies kann dadurch erzielt werden, dass der Anteil an Berylliumoxid und/oder Lithiumtetraborat in dem Stoffverbund ausreichend hoch gewählt ist, um eine stärker abweichende effektive Kernladungszahl anderer Bestandteile auszugleichen. Mit anderen Worten kann eine effektive Kernladungszahl des Stoffverbunds in dem Intervall zwischen 6,1 und 8,1, vorzugsweise zwischen 6,7 und 7,5, dadurch gewährleistet sein, dass der Anzahl an Berylliumoxid und/oder Lithiumtetraborat ausreichend hoch ist. Durch eine Wahl der effektiven Kernladungszahl in dem genannten Intervall können die eingangs genannten Vorteile für eine entsprechende Kernladungszahl in der Nähe der effektiven Kernladungszahl menschlichen Gewebes besonders vorteilhaft ausfallen.

Gemäß einer Weiterbildung ist vorgesehen, dass die Partikel des Konvertermaterials und/oder des Detektormaterials eine Korngröße von weniger als 30 Mikrometer, vorzugsweise weniger als 10 Mikrometer, aufweisen. Mit anderen Worten liegen das Konvertermaterial und/oder das Detektormaterial jeweils in Partikeln vor, deren Korngröße kleiner ist als 30 Mikrometer, vorzugsweise kleiner ist als 10 Mikrometer. Als Korngröße kann dabei beispielsweise ein Durchmesser, eine diagonale oder eine maximale Ausdehnung des entsprechenden Partikels in beliebiger Richtung gewählt sein. Durch eine entsprechende Partikelgröße können die Vorteile der Durchmischung von Konvertermaterial und Detektormaterial in dem Stoffgemenge weiter verbessert werden. Insbesondere kann die Wegstrecke, welche die Sekundärstrahlung vom Ort ihrer Entstehung, d.h. im Konvertermaterial bis zur Detektion im Detektormaterial zurücklegen muss, weiter verringert werden.

Gemäß einer Weiterbildung ist vorgesehen, dass der Stoffverbund eine ebene Oberfläche sowie eine Ausdehnung zwischen 0,2 Millimetern und 0,5 Millimetern, insbesondere eine Ausdehnung von 0,3 Millimetern, senkrecht zur Oberfläche aufweist. Mit anderen Worten kann vorgesehen sein, dass der Stoffverbund senkrecht zu der ebenen Oberfläche eine Dicke zwischen 0,2 Millimetern und 0,5 Millimetern, insbesondere eine Dicke von 0,3 Millimetern, aufweist. Vorzugsweise handelt es sich bei dem Stoffverbund um ein zylindrisches oder quaderförmiges Gebilde mit einer Höhe von 0,2 Millimetern bis 0,5 Millimetern, insbesondere mit einer Höhe von 0,3 Millimetern. Auf diese Weise ist einerseits eine ausreichende mechanische Stabilität des Stoffverbunds gewährleistet. Andererseits wäre eine zu große Dicke hinderlich bei einer späteren Auswertung mittels optisch stimulierter Lumineszenz, welche eine Durchleuchtung des gesamten Detektors mit Stimulationslicht erfordert. Typischerweise sind Detektoren nur teilweise transparent. Die genannte Dicke hat sich als vorteilhafter Kompromiss zwischen Auswertbarkeit und mechanischer Stabilität erwiesen.

Gemäß einer Weiterbildung ist vorgesehen, dass das Konvertermaterial und das Detektormaterial durch Brennen oder Sintern zu dem Stoffverbund zusammengefügt sind. Mit anderen Worten werden das Konvertermaterial und das Detektormaterial als lose Partikel in dem Stoffgemenge zusammengeführt. Anschließend ist der Stoffverbund durch das Brennen oder Sintern zusammengefügt. Insbesondere handelte es sich bei dem das Brennen oder Sintern um so genanntes isostatisches Heißpressen. Auf diese Weise kann eine gute Durchmengung von Konvertermaterial und Detektormaterial sowie eine hohe Festigkeit des Stoffverbunds gewährleistet werden.

Ein weiterer Aspekt der vorliegenden Erfindung betrifft ein Dosimeter welches als Detektoreinheit den erfindungsgemäßen Stoffverbund in mindestens einer Ausführung enthält. Insbesondere weist das Dosimeter mindestens eine Detektoreinheit mit dem erfindungsgemäßen Stoffverbund und mindestens eine weitere Detektoreinheit ohne Konvertermaterial auf. Durch den Verzicht auf das Konvertermaterial soll der zusätzliche Stoffverbund nicht empfindlich auf freie Neutronen sein, wie dies z.B. bei Berylliumoxid der Fall ist. Auf diese Weise kann der weitere Stoffverbund zur Bestimmung eines Referenzwerts für eine Photonenstrahlung, die parallel zu den eintreffenden Neutronen vom Dosimeter absorbiert wird, dienen. Die Detektoreinheit mit dem erfindungsgemäßen Stoffverbund detektiert sowohl die Neutronenstrahlung als auch die Photonenstrahlung. Die weitere Detektoreinheit detektiert die Photonenstrahlung, nicht jedoch die Neutronenstrahlung. Es kann bei der späteren Auswertung für beide Detektoreinheiten ein jeweiliger Dosiswert bestimmt werden. Nachfolgend kann die Photonenstrahlung, welche zum Teil eben auch durch das erfindungsgemäße Dosimeter detektiert wird, bestimmt werden. Durch Subtraktion des Referenzwerts, also der Photonendosis, vom Dosiswert aus der Detektoreinheit mit dem erfindungsgemäßen Stoffverbund kann die reine Neutronendosis bestimmt werden.

Ein weiterer Aspekt der vorliegenden Erfindung betrifft ein Verfahren zum Detektieren freier Neutronen, unter anderem mit den Schritten:
- zumindest teilweises Absorbieren der Neutronen durch einen Stoffverbund, in welchem ein Konvertermaterial und ein Detektormaterial jeweils in einer Vielzahl an Partikeln in einem Stoffgemenge vorliegen,
- Erzeugen einer Sekundärstrahlung durch das Konvertermaterial als Konsequenz eines Neutroneneinfangs, und
- Speichern der Sekundärstrahlungsquantität durch ein Detektormaterial aus Berylliumoxid, welches dazu ausgebildet ist, die Information bei einer späteren Auswertung mittels optisch stimulierter Lumineszenz freizugeben beziehungsweise zu quantifizieren.

Der Stoffverbund, das Dosimeter sowie die Auswertung des Stoffverbunds mittels optisch stimulierter Lumineszenz wurden bereits ausgeführt. Das vorliegende Verfahren zum Detektieren freier Neutronen wird zusätzlich durch die entsprechenden Merkmale weitergebildet, welche somit ebenso für das Verfahren gelten. Die jeweiligen Vorteile gelten dann analog.

Das Speichern erfolgt insbesondere remanent. Als Information wird insbesondere die kumulierte Quantität der Sekundärstrahlung gespeichert beziehungsweise ein Wert proportional hierzu. Das Detektormaterial ist vorzugsweise dazu ausgebildet, die gespeicherte Information bei einer späteren Auswertung dosisproportional in Form von Lumineszenzlicht abzugeben.

Die Erfindung beinhaltet weiterhin die nachfolgenden zusätzlichen Schritte zur Auswertung der Information:
- Beleuchten des Stoffverbunds mit Licht eines ersten Stimulationsspektrums, wobei das Stimulationsspektrum zur Stimulation von Berylliumoxid und Lithiumtetraborat spezifisch geeignet ist, und
- Detektieren der Neutronen anhand eines Emissionsspektrums, welches durch den Stoffverbund auf die Beleuchtung mit dem Stimulationsspektrums hin emittiert wird, entsprechend der vorbestimmten Vorschrift.

Die Auswertung des Stoffverbunds mittels optisch stimulierter Lumineszenz wurde im Kontext des Stoffverbunds bereits ausgeführt. Das vorliegende Verfahren zum Detektieren freier Neutronen wird zusätzlich durch die entsprechenden Merkmale weiter gebildet. Die jeweiligen Vorteile gelten dann analog. Die beiden Verfahrensschritte des Beleuchtens und Detektierens werden insbesondere zeitgleich durchgeführt. Insbesondere wird die gespeicherte Information bei dem vorliegenden Verfahren dosisproportional in Form von Lumineszenzlicht abgegeben.

Erfindungsgemäß für das Verfahren zum Detektieren freier Neutronen ist vorgesehen, dass
- der Stoffverbund Berylliumoxid als Detektormaterial sowie Lithiumtetraborat als Konvertermaterial beinhaltet, und
- das Beleuchten des Stoffverbunds mit zwei unterschiedlichen Stimulationsspektren erfolgt, wobei erstes der beiden Stimulationsspektren für Berylliumoxid und das andere der beiden Stimulationsspektren für Lithiumtetraborat spezifisch zur Anregung der optisch stimulierten Lumineszenz geeignet ist.

Mit anderen Worten sieht das Verfahren eine doppelte beziehungsweise zweistufige Auswertung mittels optisch stimulierter Lumineszenz vor. Im Rahmen der zweifachen Auswertung wird die jeweils im Berylliumoxid sowie dem Lithiumtetraborat gespeicherte Information insbesondere nacheinander oder gleichzeitig freigegeben. Dabei wird der Stoffverbund nacheinander oder gleichzeitig mit zwei unterschiedlichen Stimulationsspektren beleuchtet. Die beiden unterschiedlichen Stimulationsspektren können jeweils durch monochromatisches Licht unterschiedlicher Wellenlänge bereitgestellt sein. Die jeweiligen Stimulationsspektren beziehungsweise die jeweiligen Wellenlängen des monochromatischen Lichts können spezifisch für das Berylliumoxid und das Lithiumtetraborat gewählt sein. Insbesondere ist das erste Stimulationsspektrum so gewählt, dass durch dieses ausschließlich das Berylliumoxid zur optisch stimulierten Lumineszenz angeregt wird und das zweite Stimulationsspektrum so gewählt, dass durch dieses ausschließlich das Lithiumtetraborat zur optisch stimulierten Lumineszenz angeregt wird. Das Beleuchten mit dem erste Stimulationsspektrum und dem zweiten Stimulationsspektrum kann gleichzeitig, nacheinander oder zeitlich überlappend erfolgen. In Reaktion auf die jeweilige Beleuchtung geben das Lithiumtetraborat und das Berylliumoxid gleichzeitig, nacheinander oder zeitlich überlappend die jeweils gespeicherte Information frei. Dies erfolgt durch das Emittieren des jeweiligen materialspezifischen Emissionsspektrums durch das Berylliumoxid und das Lithiumtetraborat. Die beiden Emissionsspektren können getrennt detektiert werden bzw. voneinander unterschieden werden. Ein Auslesen der beiden Materialen kann durch Unterscheidung der jeweiligen Emissionsspektren unabhängig voneinander erfolgen. Anschließend können die unabhängig bestimmten Werte zusammengefasst werden. Insgesamt ergibt sich durch diese Ausführungsform eine doppelte Auswertung der Information und somit eine unabhängige Detektion der freien Neutronen durch das Berylliumoxid und das Lithiumtetraborat. Auf diese Weise kann eine deutlich erhöhte Genauigkeit gewährleistet werden.

Im Folgenden wird die Erfindung anhand von Zeichnungen konkreter Ausführungsbeispiele weiter erläutert. Die gezeigten Ausführungsbeispiele sind dabei rein beispielhaft zu verstehen und beschränken die Erfindung nicht.

Es zeigen:
- Fig. 1: ein Dosimeter, das zwei Detektoreinheiten enthält, in einer schematischen Vorderansicht;
- Fig. 2: einen Stoffverbund für eine Detektoreinheit in einer schematischen Perspektivansicht; und
- Fig. 3: ein Ablaufdiagramm eines beispielhaften Verfahrens zum Auswerten einer Neutronendosis.

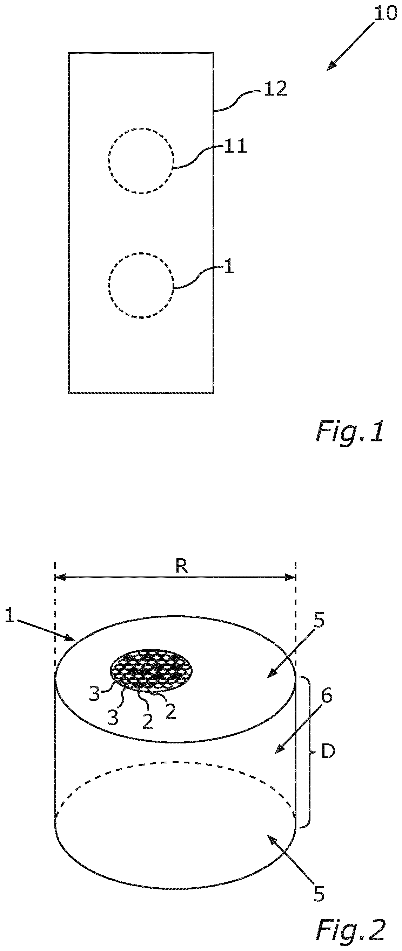

Fig. 1 zeigt ein Dosimeter 10, welches ein Gehäuse 12 aufweist. Innerhalb des Gehäuses 12 sind zwei Detektoreinheiten angeordnet. Eine erste der zwei Detektoreinheiten ist durch einen Stoffverbund 1 bereitgestellt. Die andere der zwei Detektoreinheiten wird als weitere Detektoreinheit 11 bezeichnet. Dabei ist das Dosimeter 10 dazu ausgebildet, Neutronenstrahlung, insbesondere so genannte freie Neutronen und/oder thermische Neutronen, zu erfassen. Zum Erfassen der freien Neutronen ist insbesondere der Stoffverbund 1 ausgebildet. Die weitere Detektoreinheit 11 ist hingegen dazu ausgebildet, eine Photonenstrahlung (Gamma-Strahlung, kosmische Strahlung, Röntgen-Strahlung, etc.) zu erfassen. Im Rahmen einer späteren Auswertung kann ein Referenzwert bezüglich der erfassten Photonenstrahlung bestimmt werden. Mittels dieses Referenzwerts kann durch den Stoffverbund 1 erfasste Photonenstrahlung subtrahiert werden, sodass als Auswerteergebnis allein die durch den Stoffverbund 1 detektierte Neutronendosis erhalten bleibt. Diese spätere Auswertung wird jedoch im Folgenden noch genauer erörtert.

Fig. 2 zeigt den Stoffverbund 1 in einer schematischen Perspektivansicht. Der Stoffverbund 1 hat vorliegend beispielhafterweise eine Formgebung ähnlich einer Tablette. Mit anderen Worten ist der Stoffverbund 1 vorliegend rein beispielhaft gemäß einem Zylinder geformt. Der Stoffverbund 1 weist vorliegend zwei ebene Oberflächen 5 auf. Im vorliegenden Beispiel sind die ebenen Oberflächen 5 zudem parallel zueinander. Im vorliegenden Beispiel einer zylindrischen Formgebung sind die ebenen Oberflächen 5 durch Boden- und Deckfläche des Zylinders bereitgestellt. Zwischen den ebenen Oberflächen 5 erstreckt sich im vorliegenden Beispiel die Zylindermantelfläche 6. Senkrecht zu einer oder beiden der Oberflächen 5 weist der Stoffverbund eine Dicke D auf. Im vorliegenden Beispiel sind die ebenen Oberflächen 5 jeweils kreisförmig geformt, wobei ein jeweiliger den Oberflächen 5 zugrunde liegender Kreis einen Durchmesser R aufweist.

Der Stoffverbund 1 weist ein Konvertermaterial 2 auf, welches dazu ausgebildet ist, auf einen Einfang freier Neutronen hin eine Sekundärstrahlung zu erzeugen. Ein geeignetes Konvertermaterial stellen insbesondere chemische Verbindungen (beansprucht wird lediglich Lithiumtetraborat) dar, welche das Isotop ⁶Li beinhalten. ⁶Li reagiert auf den Einfang freier Neutronen zeitnah mit einem radioaktiven Zerfall, bei dem kurzreichweitige Alphastrahlung sowie ein Tritium Partikel frei werden. In dem Konvertermaterial ist dementsprechend vorgesehen, dass das Isotop ⁶Li gegenüber seiner natürlichen Häufigkeit angereichert ist. Selbstverständlich kommen beliebige Materialien als Konvertermaterial 2 in Frage, sofern sie über nennenswerte Einfangquerschnitte für Neutronen verfügen. Unterschiedliche Materialien können dabei auch unterschiedliche Sekundärstrahlung erzeugen. Quelle der Sekundärstrahlung ist jedoch häufig eine durch den Neutroneneinfang hervorgerufene Kernreaktion. Mit anderen Worten zeichnet sich das Konvertermaterial 2 dadurch aus, dass dieses Atome enthält, die auf einen Neutroneneinfang hin radioaktiv zerfallen unter der Aussendung der Sekundärstrahlung. Dabei ist sicherzustellen, dass die Sekundärstrahlung in einer für den jeweiligen Anwendungszweck angemessenen Zeitspanne erzeugt wird. Vorteilhafterweise weist das Konvertermaterial 2 beziehungsweise im Konvertermaterial 2 enthaltene Isotope, die zum Einfangen der Neutronen und zum Erzeugen der Sekundärstrahlung ausgebildet sind, einen möglichst großen Einfangquerschnitt für Neutronen auf. Das Isotop ⁶Li weist einen ausreichend hohen Einfangquerschnitt für Neutronen auf.

Der Stoffverbund 1 umfasst weiterhin ein Detektormaterial 3, welches dazu ausgebildet ist, die Quantität der Sekundärstrahlung zu speichern und bei einer späteren Auswertung mittels optisch stimulierter Lumineszenz bestimmen zu lassen. Das Detektormaterial ist insbesondere ein Material, welches durch Speicherung freier Ladungsträger in stabilen Energieniveaus die Dosisinformation zu konservieren. Beispielsweise handelt es sich bei den Traps, die freie Ladungsträger aufnehmen können, um Energieniveaus, welche zwischen Valenzband und Leitungsband des Detektormaterials 3 liegen. Ein Zurückkehren in das Valenzband oder eine Anhebung in das Leitungsband sind von diesem Energieniveau aus nicht ohne weiteres möglich. Auf diesem Prinzip beruht die Tatsache, dass das Elektron auf dem entsprechenden Energieniveau gefangen ist und erst durch weitere Energiezufuhr befreit werden kann. Im Rahmen der späteren Auswertung mittels optisch stimulierter Lumineszenz kann durch eine entsprechende Energiezufuhr auf ein nochmals höheres Energieniveau gehoben. Beim Zurückkehren von diesem nochmals erhöhten Energieniveau auf den Grundzustand oder ein anderes niedrigeres Energieniveau, wird dann eine charakteristische Lichtabgabe erzeugt, deren Wellenlänge der frei werdenden Energie entspricht. Dies wird im Folgenden noch genauer erläutert.

Zur Anwendung im Rahmen der Personendosimetrie ist vorliegend vorgesehen, dass der Stoffverbund 1 eine effektive Kernladungszahl aufweist, welche sehr ähnlich der effektiven Kernladungszahl menschlichen Gewebes ist. Auf diese Weise können Messergebnisse, welche mittels des Stoffverbunds 1 erhalten werden, in besonderem Maße auf den menschlichen Körper beziehungsweise auf eine Person, welche das Dosimeter 10 zur Überwachung der Strahlenexposition am Körper trägt, übertragen werden. Mit anderen Worten können durch einen derartigen Stoffverbund 1 Ergebnisse in Bezug auf die Personendosimetrie erhalten werden, welche gegenüber dem Stand der Technik verbessert sind. Beispielsweise kann eine effektive Kernladungszahl im Stoffverbund 1 zwischen 6,1 und 8,1, vorzugsweise zwischen 6,7 und 7,5, vorgesehen sein.

Um eine effektive Kernladungszahl, die den oben genannten Anforderungen genügt, zu erhalten, ist gemäß der beanspruchten Erfindung vorgesehen, dass das Detektormaterial 3 aus Berylliumoxid gebildet ist. Die effektive Kernladungszahl von Berylliumoxid (BeO) ist in guter Näherung (effektive Kernladungszahl ist 7,1) äquivalent mit der effektiven Kernladungszahl von Körpergewebe. Somit findet Strahlungstransport im Berylliumoxid unter ähnlichen Bedingungen statt wie im menschlichen Körper. Der Stoffverbund 1 kann somit ohne zusätzliche Filter genutzt werden, um eine Dosis über einem weiteren Energiebereich zu erfassen.

Berylliumoxid zeichnet sich zudem dadurch aus, dass ein so genanntes Fading, also der Verlust von Dosisinformation über die Zeit, praktisch vernachlässigt werden kann. Zudem ist eine typische Detektorempfindlichkeit von Berylliumoxid so hoch, dass reproduzierbare Messungen bis in den Dosisbereich weniger Mikrosievert möglich sind. Berylliumoxid wird in nennenswerter Menge für Anwendungen als thermisch gut leitfähiger Isolator zum Beispiel in Zündkerzen eingesetzt und ist daher als Ausgangsmaterial auch für eine Anwendung in der Dosimetrie ohne weiteres verfügbar. Als Keramikmaterial ist Berylliumoxid chemisch und mechanisch sehr stabil und nicht hygroskopisch. Berylliumoxid besitzt eine Empfindlichkeit für einfallende Photonenstrahlung (Röntgen, Gamma) als auch Elektronen (Betastrahlung) und Heliumkerne (Alphastrahlung) soweit diese Teilchen in das Berylliumoxid, also vorliegend das Detektormaterial 3, eindringen können. In Reinform weist das Detektormaterial 3, also vorliegend das Berylliumoxid jedoch keine oder nur eine geringe Empfindlichkeit für die Bestrahlung mit Neutronen (thermisch oder hochenergetisch) auf. Aus diesem Grund ist die Beimischung des Konvertermaterials 2 vorgesehen, um die Sekundärstrahlung zu erzeugen, welche dann wiederum mit Hilfe des Detektormaterials 3 nachweisbar ist.

Eine andere Möglichkeit, die effektive Kernladungszahl der effektiven Kernladungszahl menschlichen Gewebes anzunähern, liegt darin, dass Konvertermaterial 2 aus Lithiumtetraborat zu bilden. Lithiumtetraborat (Li₂B₄O₇) kann sogar zwei mögliche Isotope mit einem hohen Einfangquerschnitt für Neutronen enthalten, nämlich ⁶Li und ¹⁰B. Lithiumtetraborat ist hinsichtlich seiner effektiven Kernladungszahl gewebeäquivalent zum menschlichen Körpergewebe. Um die Effizienz als Konvertermaterial 2 zu verbessern, kann das Isotop ⁶Li gegenüber anderen Lithium-Isotopen angereichert sein und/oder das Isotop ¹⁰Bor gegenüber anderen Bor-Isotopen angereichert sein.

Die folgende erste, zweite und vierte Ausführungsform sind nicht erfindungsgemäß und dienen lediglich der Veranschaulichung. Es kann also gemäß einer nicht beanspruchten ersten Ausführungsform vorgesehen sein, in dem Stoffverbund 1 Lithiumtetraborat als Konvertermaterial 2 mit einem beliebigen Detektormaterial 3, welches optisch stimulierte Lumineszenz ermöglicht, zu kombinieren. Alternativ ist es gemäß einer nicht beanspruchten zweiten Ausführungsform möglich, Berylliumoxid als Detektormaterial 3 mit einem beliebigen Konvertermaterial 2, welches dazu ausgebildet ist, auf einen Einfall freier Neutronen hin eine Sekundärstrahlung zu erzeugen, zu kombinieren. Dabei ist jeweils darauf zu achten, dass die Anteile des Berylliumoxids beziehungsweise des Lithiumtetraborats in dem Stoffverbund ausreichend groß sind, um die mittlere effektive Kernladungszahl des gesamten Stoffverbunds 1 auf einen Wert zu verschieben, der maximal um ein vorbestimmtes Maß von effektiven Kernladungszahlen menschlichen Gewebes abweicht. Beispielsweise ist der Anteil an Berylliumoxid oder der Anteil an Lithiumtetraborat in dem Stoffverbund 1 so hoch zu wählen, dass sich eine effektive Kernladungszahl für den gesamten Stoffverbund 1 zwischen 6,1 und 8,1, vorzugsweise zwischen 6,7 und 7,5, ergibt.

Im Allgemeinen ist vorgesehen, dass der Stoffverbund 1 jeweils zumindest 10 Prozent des Konvertermaterials 2 und des Detektormaterials 3 aufweist. Vorteilhafterweise weist das Detektormaterial 3 einen Anteil von mehr als 10 Prozent, beispielsweise zumindest 20 Prozent, zumindest 30 Prozent, zumindest 50 Prozent oder zumindest 70 Prozent, auf um bei der späteren Auswertung eine ausreichende Intensität der Lumineszenz zu erhalten. Auf diese Weise ist einerseits eine ausreichende Konversion der Neutronen und andererseits eine ausreichende Speicherung der Sekundärstrahlung gewährleistet.

Eine effektive Kernladungszahl, welche eine besonders hohe Gewebeäquivalenz aufweist, ergibt sich stets dann, wenn als Konvertermaterial 2 Lithiumtetraborat und als Detektormaterial 3 Berylliumoxid verwendet wird. Es kann somit gemäß einer erfindungsgemäßen dritten Ausführungsform vorgesehen sein, dass sowohl das Konvertermaterial 2 als auch das Detektormaterial 3 Gewebeäquivalenz bezüglich der jeweiligen effektiven Kernladungszahl aufweisen. In diesem Fall ist es möglich, Lithiumtetraborat als Konvertermaterial 2 mit Berylliumoxid als Detektormaterial 3 in dem Stoffverbund 1 zu kombinieren.

Dadurch, dass der Stoffverbund Berylliumoxid als Detektormaterial 3 und Lithiumtetraborat als Konvertermaterial 2 aufweist, ist die effektive Kernladungszahl unabhängig von den jeweiligen Gewichtsanteilen mit der effektiven Kernladungszahl menschlichen Gewebes äquivalent und der Volumenanteil des Konvertermaterials frei wählbar. Selbstverständlich ergeben sich diese Vorteile auch dann, wenn ein anderes Konvertermaterial 2 als Lithiumtetraborat und/oder ein anderes Detektormaterial 3 als Berylliumoxid mit einer vergleichbaren effektiven Kernladungszahl gewählt werden.

Gemäß einer nicht beanspruchten vierten Ausführungsform kann es vorgesehen sein, dass das Lithiumtetraborat sowohl als Konvertermaterial 2 als auch als Detektormaterial 3 eingesetzt wird. Dies ist darin begründet, dass Lithiumtetraborat ebenfalls das Speichern einer Information bezüglich der Sekundärstrahlung sowie eine spätere Freigabe dieser Information mittels optisch stimulierter Lumineszenz ermöglicht. Mit anderen Worten kann das Lithiumtetraborat bei einer Anwendung als Konvertermaterial 2 und Detektormaterial 3 einerseits die Sekundärstrahlung auf das Einfangen der Neutronen hin erzeugen und ebenfalls eine Information bezüglich der Sekundärstrahlung selbst speichern. Dabei erfolgen das Einfangen der Neutronen sowie das Erzeugen der Sekundärstrahlung insbesondere durch die im Lithiumtetraborat enthaltenen ⁶Li und/oder ¹⁰B Atome. Das Speichern der Information bezüglich der Sekundärstrahlung hingegen erfolgt im Wesentlichen durch den chemischen Verbund des Lithiumtetraborats.

Aufgrund der teilweise geringen Reichweite der Sekundärstrahlung und/oder um eine Abschwächung der Sekundärstrahlung auf dem Weg vom Konvertermaterial 2 zum Detektormaterial 3 zu vermeiden, ist vorliegend vorgesehen, dass das Konvertermaterial 2 und das Detektormaterial 3 jeweils in einer Vielzahl an Partikeln vorliegen, welche in dem Stoffverbund 1 gemeinsam als Stoffgemenge vorliegen. Dies ist in Fig. 2 schematisch dargestellt. Mit anderen Worten liegen das Konvertermaterial und/oder das Detektormaterial 3 jeweils in einer Vielzahl an Partikeln vor. Die jeweiligen Partikel des Konvertermaterials 2 und des Detektormaterials 3 sind untereinander in dem Stoffgemenge 1 vermischt. Auf diese Weise kann die Weglänge, welche die Sekundärstrahlung vom Konvertermaterial 2 zum Detektormaterial 3 zurücklegen muss, minimiert werden. Dies gilt insbesondere dann, wenn die jeweiligen Partikel, in welchen das Konvertermaterial 2 und/oder das Detektormaterial 3 vorliegt, eine Korngröße von weniger als 30 Mikrometern, vorzugsweise weniger als 10 Mikrometer, aufweisen.

Der Stoffverbund 1 kann beispielsweise durch Pressen, Brennen und/oder Sintern hergestellt werden. Im vorliegenden Ausführungsbeispiel ist der Stoffverbund 1 durch heiß-isostatisches Verpressen hergestellt. Ausgangsmaterial hierfür sind das Konvertermaterial 2 sowie das Detektormaterial 3 jeweils in Pulverform. Wie oben beschrieben, sind die jeweiligen Korngrößen der Partikel ein möglicher Freiheitsgrad bei der Herstellung. Der Stoffverbund 1 kann zudem optionaler Weise ein Bindemittel enthalten um den Zusammenhalt der einzelnen Partikel zu verbessern. Nach dem heißisostatischen Verpressen kann durch Brennen bei hoher Temperatur eine stabile Keramik erzeugt werden. Freiheitsgerade um die Herstellung zu optimieren bestehen dabei in Temperaturen, Temperaturprofilen und der Brenndauer. Brenntemperaturen können dabei beispielsweise bei etwa 1500 Grad Celsius liegen. Eventuell beim Pressen eingesetzte Bindemittel können sich beim Brennen mit derartigen Temperaturen zumindest teilweise zersetzen. Durch das Verpressen und das anschließende Brennen entsteht eine stabile Keramik. Der Stoffverbund 1 ist mechanisch stabil und inert. Insbesondere ist der Stoffverbund 1 sehr stabil gegenüber Abrieb. Zudem ergibt sich ein Stoffverbund 1, der chemisch sehr stabil ist. Auch ist der Stoffverbund 1 nach der entsprechenden Behandlung nicht hygroskopisch, zieht also kein Wasser an.

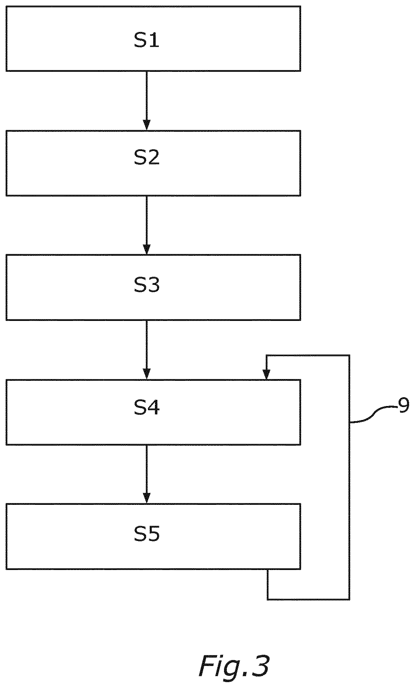

Zuletzt zeigt Fig. 3 ein Verfahren zum Detektieren freier Neutronen. Das Verfahren zum Detektieren freier Neutronen mit den Schritten S1 bis S5 beinhaltet dabei ein Verfahren zum Erfassen freier Neutronen, welches die Schritte S1 bis S3 umfasst. In einem ersten Schritt S1 wird der Stoffverbund 1 freien Neutronen ausgesetzt. Dabei wird zumindest ein Teil der freien Neutronen durch den Stoffverbund 1 absorbiert.

In einem Schritt S2 wird durch das Konvertermaterial 2 eine Sekundärstrahlung auf die Anwesenheit der Neutronen hin erzeugt. Insbesondere werden die Neutronen durch das Konvertermaterial 2 zumindest teilweise unter Erzeugung der Sekundärstrahlung eingefangen. In einem weiteren Schritt S3 wird eine Information bezüglich der Sekundärstrahlung durch das Detektormaterial 3 gespeichert. Das Detektormaterial 3 ist weiterhin dazu ausgebildet, die Information bezüglich der Sekundärstrahlung bei einer späteren Auswertung mittels optisch stimulierter Lumineszenz wieder freizugeben.

Es ist zu beachten, dass die Schritte S1, S2 und S3 in der Realität üblicherweise zeitlich überlappend oder gar zeitgleich ablaufen.

Bei der Durchführung dieses Verfahrens kann in nicht beanspruchten Ausführungsformen vorgesehen sein, dass entweder das Konvertermaterial aus Lithiumtetraborat gebildet ist oder das Detektormaterial aus Berylliumoxid gebildet ist. Alternativ kann erfindungsgemäß vorgesehen sein, dass das Konvertermaterial 2 aus Lithiumtetraborat und gleichzeitig das Detektormaterial 3 aus Berylliumoxid gebildet sind. Gemäß einer weiteren nicht beanspruchten Alternative kann vorgesehen sei, dass sowohl das Konvertermaterial 2 als auch das Detektormaterial 3 aus Lithiumtetraborat gebildet sind.

Die spätere Auswertung kann im Wesentlichen durch die weiteren Schritte S4 und S5 gegeben sein. In einem Schritt S4 wird der Stoffverbund 1 mit Licht eines Stimulationsspektrums beleuchtet. Dabei ist das Stimulationsspektrum für das Detektormaterial 3, also Berylliumoxid, spezifisch zur Stimulation geeignet. Insbesondere handelt es sich bei dem Stimulationsspektrum um zumindest im Wesentlichen monochromatisches Licht, wobei eine Wellenlänge des zumindest im Wesentlichen monochromatischen Lichts spezifisch für das Detektormaterial 3, also Berylliumoxid ist. Spezifisch bedeutet insbesondere, dass eine Energie von Photonen des Stimulationsspektrums ausreicht, um Elektronen aus den Traps zu befreien. In anderen Schritt S5 wird insbesondere gleichzeitig zum Schritt S4 ein Emissionsspektrum detektiert, welches durch den Stoffverbund 1, insbesondere das Detektormaterial 3, auf die Beleuchtung mit dem Stimulationsspektrum hin emittiert wird. Dabei kann gemäß einer vorbestimmten Vorschrift eine Intensität der Neutronen aus der Intensität des Emissionsspektrums abgeleitet werden. Insbesondere wird eine Neutronendosis aus der Anzahl an Photonen des Emissionsspektrums bestimmt. Beispielsweise kann die festgestellte Neutronendosis gemäß der vorbestimmten Vorschrift proportional zu der Anzahl detektierter Photonen des Emissionsspektrums sein. Bei den Photonen des Emissionsspektrums handelt es sich insbesondere um monochromatisches Licht einer zweiten Wellenlänge. Die zweite Wellenlänge ist insbesondere spezifisch für das Detektormaterial 3, nämlich Berylliumoxid.

Die Schritte S4 und S5 werden insbesondere gleichzeitig durchgeführt. Dies kann darauf zurückzuführen sein, dass das Detektormaterial zumindest annähernd instantan mit der Emission des Emissionsspektrums auf die Stimulation mit dem Stimulationsspektrum reagiert. Um keine Dosisinformation zu verlieren, ist es jedoch auch nötig, das Detektieren gemäß S5 während der gesamten Dauer der Emission des Emissionsspektrums durchzuführen.

Zuletzt kann im Rahmen des vorliegenden Verfahrens eine Schleife 9 durchlaufen werden, sodass die Schritte des Beleuchtens des Stoffverbunds und des Detektierens der Neutronen, also die Schritte S4 und S5, mehrfach durchgeführt werden. Dabei ist vorgesehen, dass das Beleuchten des Stoffverbunds nacheinander oder gleichzeitig mit zwei unterschiedlichen Stimulationsspektren erfolgt. Dies ist bei der beanspruchten Erfindung vorgesehen, da das Konvertermaterial 2 aus Lithiumtetraborat und das Detektormaterial 3 aus Berylliumoxid gebildet ist. Denn wie oben bereits beschrieben, sind in diesem Fall zwei unterschiedliche Materialien, welche eine Auswertung mittels optisch stimulierter Lumineszenz ermöglichen, in dem Stoffverbund 1 vorhanden. Dementsprechend ist es vorgesehen, dass in dem Schritt S4 der Stoffverbund 1 nacheinander oder gleichzeitig mit den zwei unterschiedlichen Stimulationsspektren beleuchtet wird, wobei ein erstes der zwei unterschiedlichen Stimulationsspektren für Berylliumoxid spezifisch ist und das andere der beiden Stimulationsspektren für Lithiumtetraborat spezifisch ist. Auf analoge Weise werden dann zwei unterschiedliche Emissionsspektren detektiert, wobei ein erstes der Emissionsspektren für Berylliumoxid und das andere der beiden Emissionsspektren für Lithiumtetraborat spezifisch sein können. Da sich sowohl die Stimulationsspektren als auch die Emissionsspektren jeweils untereinander unterscheiden können, ist es möglich, das Beleuchten mit den beiden Stimulationsspektren sowie das Detektieren der beiden Emissionsspektren gleichzeitig durchzuführen. Durch die jeweils unterschiedlichen Wellenlängen kann eine gegenseitige Beeinflussung unter Umständen ausgeschlossen werden. Alternativ ist es möglich, dass das Beleuchten des Stoffverbunds 1 mit den beiden unterschiedlichen Stimulationsspektren nacheinander durchgeführt wird. Dementsprechend wird in diesem Fall auch das Detektieren der beiden Stimulationsspektren nacheinander durchgeführt. Das Beleuchten mit dem ersten Stimulationsspektrum und das Detektieren des ersten Emissionsspektrums erfolgt gleichzeitig. Analog erfolgt das Beleuchten mit dem zweiten Stimulationsspektrum und das Detektieren des zweiten Emissionsspektrums gleichzeitig.

Im Rahmen der Auswertung kann auch der Referenzwert bezüglich der erfassten Photonenstrahlung bestimmt werden. Der Referenzwert wird mittels der weiteren Detektoreinheit 11 bestimmt. Die weitere Detektoreinheit 11 kann dem Stoffverbund 1 nachempfunden sein, jedoch weist die weitere Detektoreinheit 11 kein Konvertermaterial 2 auf. Somit weist die weitere Detektoreinheit 11 keine nennenswerte beziehungsweise nur eine geringe Empfindlichkeit für Neutronenstrahlung auf. Beispielsweise ist die Empfindlichkeit für Neutronenstrahlung der weiteren Detektoreinheit 11 verglichen mit dem Stoffverbund 1 zumindest um den Faktor 10 oder 100 geringer. Beispielsweise erfasst die weitere Detektoreinheit 11 ausschließlich ionisierende Strahlung, insbesondere die Photonenstrahlung. Mittels des Referenzwerts kann dann durch den Stoffverbund 1 erfasste Photonenstrahlung subtrahiert werden, sodass als Auswerteergebnis allein die durch den Stoffverbund 1 detektierten Neutronen erhalten werden.

### BEZUGSZEICHENLISTE:

- 1: Stoffverbund
- 2: Konvertermaterial
- 3: Detektormaterial
- 5: Oberflächen
- 6: Zylindermantelfläche
- 9: Schleife
- 10: Dosimeter
- 11: Stoffverbund
- 12: Gehäuse
- D: Dicke
- R: Durchmesser
- S1: Verfahrensschritt
- S2: Verfahrensschritt
- S3: Verfahrensschritt
- S4: Verfahrensschritt
- S5: Verfahrensschritt

## Patentansprüche

1. Stoffverbund (1) zum Detektieren freier Neutronen, mit
- einem Konvertermaterial (2), welches dazu ausgebildet ist, als Folge eines Neutroneneinfangs eine Sekundärstrahlung zu erzeugen, und
- einem Detektormaterial (3), welches dazu ausgebildet ist, eine Information bezüglich einer Quantität der Sekundärstrahlung zu speichern und bei einer späteren Auswertung mittels optisch stimulierter Lumineszenz wieder freizugeben, wobei
- das Konvertermaterial (2) und das Detektormaterial (3) jeweils in einer Vielzahl an Partikeln vorliegen, welche gemeinsam in dem Stoffverbund (1) als Stoffgemenge vorliegen,
**dadurch gekennzeichnet, dass**
das Detektormaterial (3) aus Berylliumoxid und das Konvertermaterial (2) aus Lithiumtetraborat gebildet ist.

2. Stoffverbund (1) nach Anspruch 1,
**dadurch gekennzeichnet, dass**
bei dem Lithiumtetraborat die Isotope ⁶Li und/oder ¹⁰B gegenüber ihrer natürlichen Häufigkeit angereichert sind.

3. Stoffverbund (1) nach einem der vorgehergehenden Ansprüche,
**dadurch gekennzeichnet, dass**
die Anteile an dem Konvertermaterial (2) und dem Detektormaterial (3) in dem Stoffverbund (1) derart gewählt sind, dass sich eine effektive Kernladungszahl zwischen 6,1 und 8,1, vorzugsweise zwischen 6,7 und 7,5, ergibt.

4. Stoffverbund (1) nach einem der vorgehergehenden Ansprüche,
**dadurch gekennzeichnet, dass**
die Partikel des Konvertermaterials (2) und/oder des Detektormaterials (3) eine Korngröße von weniger als 30 Mikrometer, vorzugsweise weniger als 10 Mikrometer, aufweisen.

5. Stoffverbund (1) nach einem der vorgehergehenden Ansprüche,
**dadurch gekennzeichnet, dass**
der Stoffverbund (1) eine ebene Oberfläche (5) sowie eine Ausdehnung (D) zwischen 0,2 Millimetern und 0,5 Millimetern, insbesondere eine Ausdehnung (D) von 0,3 Millimetern, senkrecht zur Oberfläche (5) aufweist.

6. Stoffverbund (1) nach einem der vorgehergehenden Ansprüche,
**dadurch gekennzeichnet, dass**
das Konvertermaterial (2) und das Detektormaterial (3) durch Brennen oder Sintern zu dem Stoffverbund (1) zusammengefügt sind.

7. Dosimeter mit einem Stoffverbund (1) nach einem der vorgehergehenden Ansprüche.

8. Verfahren zum Detektieren freier Neutronen mithilfe eines Stoffverbunds (1) mit den Schritten :
- zumindest teilweises Absorbieren der Neutronen durch einen Stoffverbund (1), in welchem ein Konvertermaterial (2) und ein Detektormaterial (3) jeweils in einer Vielzahl an Partikeln in einem Stoffgemenge vorliegen,
- Erzeugen einer Sekundärstrahlung durch das Konvertermaterial (2) in Folge eines Einfangs der Neutronen, und
- Speichern einer Information bezüglich einer Quantität der Sekundärstrahlung durch das Detektormaterial (3), welches dazu ausgebildet ist, die Information bei einer späteren Auswertung mittels optisch stimulierter Lumineszenz wieder freizugeben,
sowie den zusätzlichen Schritten zur Auswertung der Information:
- Beleuchten des Stoffverbunds (1) mit Licht eines Stimulationsspektrums, und
- Detektieren der Neutronen anhand eines Emissionsspektrum, welches durch den Stoffverbund (1) auf die Beleuchtung mit dem Stimulationsspektrum hin emittiert wird, entsprechend einer vorbestimmten Vorschrift,
**dadurch gekennzeichnet, dass**
- der Stoffverbund (1) Berylliumoxid als Detektormaterial (3) sowie Lithiumtetraborat als Konvertermaterial (2) beinhaltet, und
- das Beleuchten des Stoffverbunds (1) mit zwei unterschiedlichen Stimulationsspektren erfolgt, wobei eines der beiden Stimulationsspektren für Berylliumoxid und das andere der beiden Stimulationsspektren für Lithiumtetraborat spezifisch ist.

## Claims

1. A material composite (1) for detecting free neutrons, comprising
- a converter material (2), which is formed to generate secondary radiation as a result of neutron capture, and
- a detector material (3), which is formed to store information with respect to a quantity of the secondary radiation and to release it again upon later evaluation by means of optically stimulated luminescence, wherein
- the converter material (2) and the detector material (3) are each present in a plurality of particles, which are commonly present as a material mixture in the material composite (1),
**characterized in that**
the detector material (3) is formed of beryllium oxide and the converter material (2) is formed of lithium tetraborate.

2. The material composite (1) according to claim 1,
**characterized in that**
in the lithium tetraborate, the isotopes ⁶Li and/or ¹⁰B are enriched with respect to their natural frequency.

3. The material composite (1) according to any one of the preceding claims, **characterized in that**
the portions of the converter material (2) and the detector material (3) in the material composite (1) are selected such that an effective atomic number between 6.1 and 8.1, preferably between 6.7 and 7.5 arises.

4. The material composite (1) according to any one of the preceding claims, **characterized in that**
the particles of the converter material (2) and/or of the detector material (3) have a grain size of less than 30 micrometers, preferably less than 10 micrometers.

5. The material composite (1) according to any one of the preceding claims, **characterized in that**
the material composite (1) has a flat surface (5) as well as an extension (D) between 0.2 millimeters and 0.5 millimeters, in particular an extension (D) of 0.3 millimeters, perpendicular to the surface (5).

6. The material composite (1) according to any one of the preceding claims, **characterized in that**
the converter material (2) and the detector material (3) are joined together to the material composite (1) by firing or sintering.

7. A dosimeter with a material composite (1) according to any one of the preceding claims.

8. A method for detecting free neutrons with the aid of a material composite (1) comprising the steps:
- at least partially absorbing the neutrons by a material composite (1), in which a converter material (2) and a detector material (3) are each present in a plurality of particles in a material mixture,
- generating secondary radiation by the converter material (2) as a result of capture of the neutrons, and
- storing information with respect to a quantity of the secondary radiation by the detector material (3), which is formed to again release the information upon later evaluation by means of optically stimulated luminescence,
as well as the additional steps for evaluating the information:
- illuminating the material composite (1) with light of a stimulation spectrum, and
- detecting the neutrons based on an emission spectrum, which is emitted by the material composite (1) upon the illumination with the stimulation spectrum, corresponding to a predetermined rule,
**characterized in that**
the material composite (1) includes beryllium oxide as the detector material (3) as well as lithium tetraborate as the converter material (2), and
- illuminating the material composite (1) is effected with two different stimulation spectra, wherein one of the two stimulation spectra is specific to beryllium oxide and the other one of the two stimulation spectra is specific to lithium tetraborate.

## Revendications

1. Composite de matières (1) destiné à la détection de neutrons libres, comprenant
- un matériau convertisseur (2) conçu pour générer un rayonnement secondaire à la suite d'une capture de neutrons, et
- un matériau détecteur (3) qui est conçu pour stocker une information relative à une quantité du rayonnement secondaire et la restituer lors d'une évaluation ultérieure au moyen d'une luminescence stimulée optiquement,
- le matériau convertisseur (2) et le matériau détecteur (3) étant chacun présents dans un grand nombre de particules qui sont présentes conjointement dans le composite de matières (1) sous la forme d'un mélange de matières,
**caractérisé en ce que**
le matériau détecteur (3) est formé d'oxyde de béryllium et le matériau convertisseur (2) est formé de tétraborate de lithium.

2. Composite de matières (1) selon la revendication 1,
**caractérisé en ce que**
dans le tétraborate de lithium les isotopes ⁶Li et/ou ¹⁰B sont enrichis par rapport à leur abondance naturelle.

3. Composite de matières (1) selon l'une des revendications précédentes,
**caractérisé en ce que**
les proportions du matériau convertisseur (2) et du matériau détecteur (3) dans le composite de matières (1) sont choisies de manière à obtenir un nombre atomique effectif compris entre 6,1 et 8,1, de préférence entre 6,7 et 7,5.

4. Composite de matières (1) selon l'une des revendications précédentes,
**caractérisé en ce que**
les particules du matériau convertisseur (2) et/ou du matériau détecteur (3) ont une granulométrie inférieure à 30 micromètres, de préférence inférieure à 10 micromètres.

5. Composite de matières (1) selon l'une des revendications précédentes,
**caractérisé en ce que**
le composite de matières (1) présente une surface plane (5) et une extension (D) comprise entre 0,2 millimètre et 0,5 millimètre, notamment une extension (D) de 0,3 millimètre, perpendiculairement à la surface (5).

6. Composite de matières (1) selon l'une des revendications précédentes,
**caractérisé en ce que**
le matériau convertisseur (2) et le matériau détecteur (3) sont assemblés par calcination ou frittage pour former le composite de matières (1).

7. Dosimètre comprenant un composite de matières (1) selon l'une des revendications précédentes.

8. Procédé de détection de neutrons libres à l'aide d'un composite de matières (1), ledit procédé comprenant les étapes suivantes :
- absorption au moins partielle des neutrons par un composite de matières (1) dans lequel un matériau convertisseur (2) et un matériau détecteur (3) sont chacun présents en un grand nombre de particules dans un mélange de matières,
- génération d'un rayonnement secondaire par le matériau convertisseur (2) à la suite d'une capture des neutrons, et
- stockage d'une information relative à une quantité de rayonnement secondaire par le matériau détecteur (3) qui est conçu pour restituer l'information lors d'une évaluation ultérieure par luminescence stimulée optiquement,
ainsi que les étapes supplémentaires d'évaluation de l'information :
- illumination du composite de matières (1) avec une lumière d'un spectre de stimulation, et
- détection des neutrons à l'aide d'un spectre d'émission qui est émis par le composite de matières (1) lors de l'illumination avec le spectre de stimulation, selon une règle prédéterminée,
**caractérisé en ce que**
- le composite de matières (1) contient de l'oxyde de béryllium comme matériau détecteur (3) et du tétraborate de lithium comme matériau convertisseur (2), et
- l'illumination du composite de matières (1) est effectuée avec deux spectres de stimulation différents, l'un des deux spectres de stimulation étant spécifique à l'oxyde de béryllium et l'autre des deux spectres de stimulation étant spécifique au tétraborate de lithium.
